(19) Europäisches Patentamt
European Patent Office
Office européen des brevets

(11) Veröffentlichungsnummer: **0 247 428**
**A2**

(12) # EUROPÄISCHE PATENTANMELDUNG

(21) Anmeldenummer: **87106837.5**

(51) Int. Cl.[4]: **C07H 15/12** , C07H 15/18

(22) Anmeldetag: **12.05.87**

(30) Priorität: **23.05.86 DE 3617272**

(43) Veröffentlichungstag der Anmeldung:
**02.12.87 Patentblatt 87/49**

(84) Benannte Vertragsstaaten:
**BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Hassel, Tillmann, Dr.**
**Morgengraben 14**
**D-5000 Köln 80(DE)**
Erfinder: **Müller, Hanns Peter, Dr.**
**Weizenfeld 36**
**D-5060 Bergisch Gladbach2(DE)**
Erfinder: **Bonse, Gerhard, Dr.**
**Wolfskaul 3**
**D-5000 Köln 80(DE)**
Erfinder: **Lockhoff, Oswald, Dr.**
**Morgengraben 14**
**D-5000 Köln 80(DE)**

(54) **Isocyaniddichlorid-Gruppen aufweisende Zucker und Verfahren zu ihrer Herstellung.**

(57) Die Erfindung betrifft neue Zucker, die eine Isocyaniddichlorid-Gruppe an eines der nicht-anomeren C-Atome des Zuckermoleküls gebunden enthalten und ihre Herstellung durch Chlorieren von Zuckern, die eine Isonitrilgruppe an eines der nicht-anomeren C-Atome des Zuckermoleküls gebunden enthalten.

EP 0 247 428 A2

# Isocyaniddichlorid-Gruppen aufweisende Zucker und Verfahren zu ihrer Herstellung

Die Erfindung betrifft neue, eine Isocyaniddichlorid-Gruppe (= Dichlormethylenimino-Gruppe) aufweisende Zucker und ein Verfahren zu ihrer Herstellung.

Eine Isocyaniddichlorid-Gruppe aufweisende Zucker, die diese Gruppe an ein anomeres C-Atom des Zuckermoleküls gebunden enthalten, sind bekannt (siehe EP-A-0 171 016). Als anomere C-Atome werden in der Chemie der Kohlenhydrate die durch die intramolekulare (Halb)Acetalbildung zu asymmetrischen C-Atomen gewordenen C-Atome der Carbonylgruppen der Zuckermoleküle bezeichnet (siehe Römpps Chemie-Lexikon, 8. Auflage, "Anomere"). Diese bekannten Isocyaniddichlorid-Gruppen aufweisenden Zucker werden als wertvolle Zwischenprodukte für die Synthese biologisch aktiver Verbindungen beschrieben (siehe EP-A-0 171 016, Seite 9).

Es wurde nun gefunden, daß Zucker, in denen die Isocyaniddichlorid-Gruppe an eines der nicht-anomeren Kohlenstoff atome des Zuckermoleküls gebunden ist, ebenfalls wertvolle Zwischenprodukte für die Herstellung biologisch aktiver Verbindungen sind. Es wurde gefunden, daß sie neue und verbesserte Herstellungswege für antibiotisch und canzerostatisch wirksame Zuckerureide und deren Derivate eröffnen, die über die Zucker, die die Isocyaniddichlorid-Gruppe an ein anomeres C-Atom gebunden enthalten, nicht erhältlich sind.

So ist z.B. mit Hilfe eines der Zucker, in denen die Isocyaniddichlorid-Gruppe an eines der nicht-anomeren Kohlenstoffatome des Zuckermoleküls gebunden ist, das Breitbandantibiotikum Streptozotocin aus 2-Dichlormethylenimino-2-deoxy-1.3.4.6-tetra-O-acetyl-$\beta$-D-glucopyranose dadurch erhältlich, daß man diese Verbindung mit der äquimolaren Menge Methylamin in Gegenwart einer Hilfsbase zum Carbodiimid umsetzt, das Carbodiimid mit Wasser zum entsprechenden Harnstoff hydrolysiert, die Acetylgruppen abspaltet und das erhaltene Produkt nitrosiert.

Die erfindungsgemäßen Zucker, in denen die Isocyaniddichlorid-Gruppe an ein nicht-anomeres C-Atom des Zuckermoleküls gebunden ist, sind neu. Obwohl sie sich von den in der EP-A-0 171 016 beschriebenen Zuckern mit Isocyaniddichlorid-Gruppe nur durch die Stellung dieser Gruppe im Molekül unterscheiden, wurden sie trotzdem durch diese vorbekannten Zucker nicht nahegelegt, weil für sie erst ein Herstellungsverfahren gefunden werden mußte. Die in der EP-A-0 171 016 für die Herstellung der Zucker mit an das anomere C-Atom gebundener Isocyaniddichlorid-Gruppe beschriebene Chlorierung der O-acetylierten Zucker mit an das anomere C-Atom gebundener Isothiocyanat-Gruppe läßt sich nicht auf O-acylierte Zucker anwenden, die die Isothiocyanat-Gruppe an ein nicht-anomeres C-Atom gebunden enthalten. Die Chlorierung O-acylierter Zucker, in denen die Isothiocyanat-Gruppe an ein nicht-anomeres C-Atom gebunden ist, liefert nur ein Gemisch von Chlorierungsprodukten, aus denen sich keine Zucker mit an ein nicht-anomeres C-Atom gebundener Isocyaniddichlorid-Gruppe isolieren lassen.

Erfindungsgemäß wurde gefunden, daß Zucker, die eine Isocyaniddichlorid-Gruppe an ein nicht-anomeres C-Atom gebunden enthalten, auf einfache Weise in ausgezeichneten Ausbeuten durch schonendes Chlorieren von Zuckern, die eine Isocyanid-Gruppe an ein nicht-anomeres C-Atom gebunden enthalten, erhältlich sind.

Dieses Verfahren ist universell zur Herstellung von Zuckern anwendbar, die eine Isocyaniddichlorid-Gruppe an ein nicht-anomeres C-Atom gebunden enthalten. Es ist unabhängig von der Natur der zum Schutz der OH-Gruppen des Zuckermoleküls eingeführten Gruppen; d.h. es gelingt unabhängig davon, ob die OH-Gruppen durch Veresterung, Veretherung, Acetalisierung oder Ketalisierung geschützt sind.

Zwar ist die Herstellung von Isocyaniddichloriden durch Chlorieren von Isonitrilen bekannt (siehe Houben-Weyl, Methoden der Organischen Chemie, 4. Auflage, Band E IV, Seite 526). Die vorbekannte Chlorierung von Isonitrilen zu Isocyaniddichloriden legt aber das erfindungsgemäße Verfahren nicht nahe, weil in den bislang chlorierten Isonitrilen die Isocyanidgruppe an organische Reste gebunden ist, die bei der Chlorierung nicht angegriffen werden. Im Gegensatz dazu werden erfindungsgemäß Isocyanide chloriert, bei denen die Isocyangruppe an Zuckerreste, d.h. an Reste gebunden ist, die bekannterweise chlorierungsempfindlich sind. Es war deshalb damit zu rechnen, daß bei der Einwirkung von Chlor auf die erfindungsgemäß einzusetzenden Isonitrile eine Chlorierung der Ether-, Ester-und Acetal-Schutzgruppen sowie eine Chlorierung der Zucker selbst, vor allem an den besonders reaktiven, an das anomere C-Atom gebundenen Gruppen eintritt. Es ist bekannt, daß Ether außerordentlich empfindlich gegenüber $\alpha$-Halogenierung sind (L.Summers; Chem. Rev. 55, 301 ff (1955)); die Halogenierung durch Acetalgruppen geschützter Zucker unter Abspaltung der Schutzgruppe ist eine Standardmethode zur Herstellung von Halogenozuckern, die das Halogen an ein nicht-anomeres C-Atom des Zuckermoleküls gebunden enthalten (siehe Adv. Carbohydr. Chem. Biochem. 28, 255 ff (1973)).

Die Erfindung betrifft daher eine Isocyaniddichlorid-Gruppe aufweisende Zucker, die dadurch gekennzeichnet sind, daß sie die Isocyaniddichlorid-Gruppe an eines der nicht-anomeren C-Atome des Zuckermoleküls gebunden enthalten.

Diese erfindungsgemäßen Zucker lassen sich durch die allgemeine Formel

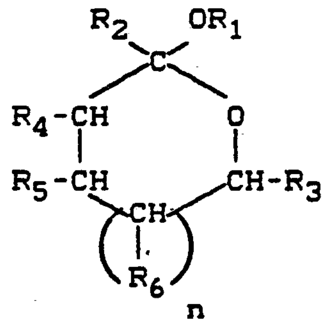

I

beschreiben, in der

n 0 oder 1,
$R_1$ eine Schutzgruppe und
$R_2$ Wasserstoff, Methyl, Ethyl, oder $-CH_2-R_7$ ist,
$R_3$ für Wasserstoff, Methyl, Ethyl, $-CH_2-R_8$ oder $-C(H)R_9-CH_2-R_{10}$ steht und
$R_4$, $R_5$, $R_6$, $R_7$, $R_8$, $R_9$ und $R_{10}$ unabhängig voneinander für Wasserstoff, Halogen, eine geschützte OH-Gruppe, eine Isocyaniddichlorid-Gruppe oder den Rest eines cyclischen Acetals der Formel

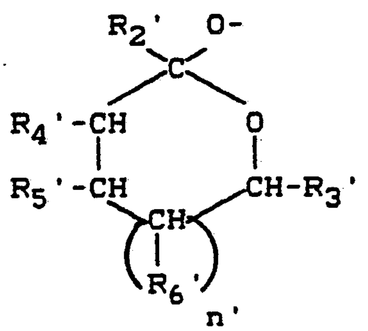

II

stehen, in der

n' 0 oder 1,
$R_2'$ Wasserstoff, Methyl, Ethyl, $-CH_2-R_7'$ ist,
$R_3'$ für Wasserstoff, Methyl, Ethyl, $-CH_2-R_8'$ oder $-C(H)R_9'-CH_2-R_{10}'$ steht, und
$R_4'$, $R_5'$, $R_6'$, $R_7'$, $R_8'$, $R_9'$ und $R_{10}'$ unabhängig voneinander für Wasserstoff, Halogen, eine geschützte OH-Gruppe oder den Rest eines cyclischen Acetals der Formel II stehen,
mit der Maßgabe,
daß nur einer der Reste $R_4$-$R_{10}$ eine Isocyaniddichlorid-Gruppe ist; daß höchstens einer der Reste $R_4$-$R_{10}$ und höchstens einer der Reste $R_4'$ -$R_{10}'$ für den Rest eines cyclischen Acetals der Formel II steht und daß mindestens einer der Reste $R_4$-$R_{10}$ und mindestens einer der Reste $R_4'$-$R_{10}'$ eine geschützte OH-Gruppe ist.

Bei den Verbindungen der Formel I handelt es sich um (Desoxy-oder Halogeno-)Zucker mit geschützten OH-Gruppen und einer an ein nicht-anomeres C-Atom gebundener Isocyaniddichlorid-Gruppe. Je nachdem, ob einer der Reste $R_4$-$R_{10}$ für den Rest eines cyclischen Acetals der Formel II steht und ob in dieser Formel II wiederum einer der Reste $R_4'$-$R_{10}'$ der Rest eines cyclischen Acetals der Formel II ist, sind diese Zucker Mono-, Di-oder Trisaccharide. Bevorzugt sind Mono-und Disaccharide, d.h. Verbindungen der Formel I die den Rest des cyclischen Acetals der Formel II höchstens einmal aufweisen.

Bevorzugt sind solche erfindungsgemäßen Zucker der Formel I, in der

n 0 oder 1
$R_1$ eine Schutzgruppe und
$R_2$ Wasserstoff oder $-CH_2-R_7$ ist,
$R_3$ für Wasserstoff, Methyl, $-CH_2-R_8$ oder $-C(H)R_9-CH_2-R_{10}$ steht und

$R_4$, $R_5$, $R_6$, $R_7$, $R_8$, $R_9$ und $R_{10}$ unabhängig voneinander Wasserstoff, eine geschützte OH-Gruppe oder eine Isoyaniddichloridigruppe bedeuten,
mit der Maßgabe, daß nur einer der Reste $R_4$-$R_{10}$ eine Isocyaniddichloridgruppe ist und mindestens zwei der Reste $R_4$-$R_{10}$ eine geschützte OH-Gruppe sind.

Besonders bevorzugt sind solche erfindungsgemäßen Zucker der Formel I, wenn in dieser
n 0 oder 1
$R_1$ eine Schutzgruppe und
$R_2$ Wasserstoff ist,
$R_3$ für Wasserstoff, $-CH_2-R_8$ oder $-C(H)R_9-CH_2-R_{10}$ steht und
$R_4$, $R_5$, $R_6$, $R_8$, $R_9$ und $R_{10}$ unabhängig voneinander eine geschützte OH-Gruppe oder eine Isocyaniddichlorid-Grupp bedeuten,
mit der Maßgabe, daß nun einer der Reste $R_4$, $R_5$, $R_6$, $R_8$, $R_9$ oder $R_{10}$ eine Isocyaniddichlorid-Gruppe ist.

Bei diesen besonders bevorzugten Zuckern der Formel I handelt es sich um Aldohexosen und -pentosen der Pyranose-und Furanose-Reihe, in denen eine der an ein nicht anomeres C-Atom gebundenen, geschützten OH-Gruppen durch eine Isocyaniddichloridgruppe ersetzt ist.

Als Halogen kommt für die Reste $R_4$-$R_{10}$ und $R_4'$-$R_{10}'$ vorzugsweise Fluor, Chlor oder Brom in Betracht.

Als "Schutzgruppen" werden in der organischen Chemie solche organischen Reste bezeichnet, mit denen bestimmte funktionelle Gruppen, im vorliegenden Fall die OH-Gruppen des Zuckers, eines mehrere reaktionsfähige Zentren enthaltenden Moleküls, hier des Zuckermoleküls, vorübergehend gegen den Angriff von Reagenzien geschützt werden können, so daß die Reaktionen nur an den gewünschten (ungeschützten) Stellen, im vorliegenden Fall den Isonitril-und Isocyaniddichlorid-Gruppen, stattfinden (siehe Römpps Chemie-Lexikon, 7, Auflage, "Schutzgruppen"). Als Schutzgruppen kommen für $R_1$ und die geschützten OH-Gruppen die aus der Zuckerchemie bekannten Schutzgruppen in Betracht; es sind dies Acylgruppen, Alkyl-und Aralkyl-Gruppen und, falls zwei benachbarte OH-Gruppen durch Acetal-oder Ketal-Bildung geschützt werden, Alkyliden-Gruppen.

Die Acylreste werden durch Acylierung der OH-Gruppen eingeführt. Die Acylreste leiten sich vorzugsweise von niederen aliphatischen, z.B. $C_1$-$C_8$-Alkancarbon-oder -sulfonsäuren wie Essigsäure, Propionsäure, Buttersäure, Methansulfonsäure und Ethansulfonsäure, oder aromatischen Carbon-und Sulfonsäuren, wie der Benzoesäure und der p-Toluolsulfonsäure ab. Bevorzugte Acylreste sind der Methansulfonyl-(mesyl)-Rest, der p-Toluolsulfonyl-(tosyl)-Rest und vor allem der Acetyl-und der Benzoyl-Rest.

Die Alkyl-und Aralkyl-Reste werden durch Veretherung der OH-Gruppen eingeführt. Als Alkyl-und Aralkylreste seien beispielsweise genannt: $C_1$-$C_8$-Alkylreste wie der Methyl-, Propyl-, Isopropyl-und der sec.-Butyl-Rest und als Aralkyl-Reste der Benzyl-, β-Phenylethyl-, der Triphenylmethyl-(trityl)-und der 4-Methylbenzyl-Rest. Bevorzugt sind niedere Alkylreste wie der Methyl-und unsubstituierte Aralkylreste wie der Benzyl-Rest.

Die Alkyliden-Reste werden durch Acetalisierung oder Ketalisierung zweier benachbarter OH-Gruppen eingeführt. Diese Alkyliden-Reste leiten sich vorzugsweise von niederen aliphatischen und aromatischen Aldehyden und Ketonen wie Formaldehyd, Acetaldehyd, Propionaldehyd, Butyraldehyd, Isobutyraldehyd, Aceton, Butanon-2, Cyclohexanon, Benzaldehyd und 4-Methoxybenzaldehyd ab. Bevorzugte Alkyliden-Reste sind: der Methyliden-, Ethyliden-, Isopropyliden-, Cyclohexyliden-, Benzyliden-und 4-Methoxybenzyliden-Rest.

Als Vertreter der erfindungsgemäßen neuen Zucker der Formel I seien beispielsweise genannt:

2-Dichlormethylenimino-2.6-di-deoxy-1.3.4-tri-O-acetyl-D-glucopyranose;
2-Dichlormethylenimino-2-deoxy-1-O-methyl-3.4.6-tri-O-benzoyl-D-mannopyranose;
2-Dichlormethylenimino-2-deoxy-1-O-methyl-3-O-methansulfonyl-4.6-O-benzyliden-D-altropyranose;
3--Dichlormethylenimino-3-deoxy-1.2-O-isopropyliden-5.6-O-cyclohexyliden-α-D-glucofuranose;
3-Dichlormethylenimino-3-deoxy-1.2-O-isopropyliden-5.6-O-methyliden-α-D-allofuranose;
2-Dichlormethylenimino-2-deoxy-1.3.4.6-tetra-O-acetyl-β-D-glucopyranose;
6-Dichlormethylenimino-6-deoxy-1,2-O-isopropyliden-3.5-di-O-acetyl-α-D-glucofuranose;
6-Dichlormethylenimino-6-deoxy-1-O-methyl-2.3.4-tri-O-acetyl-α-D-glucopyranose;
6-Dichlormethylenimino-6-deoxy-1,2-O-isopropyliden-3,5-di-O-benzyl-α-D-glycopyranose;
3-Dichlormethylenimino-3-deoxy-1,2-O-isopropyliden-5.6-O-isopropyliden-α-D-glucofuranose;
4-Dichlormethylenimino-4-deoxy-1-O-methyl-2.3.6-tri-O-benzyl-α-D-glucopyranose;
3-Dichlormethylenimino-3-deoxy-1.2-O-isopropyliden-5,6-O-isopropyliden-α-D-allofuranose sowie
6-Dichlormethylenimino-2.6-di-deoxy-2-chloro-1-O-methyl-3.4-di-O-acetyl-D-mannopyranose und
5-Dichlormethylenimino-5-deoxy-2.3-O-isopropyliden-1-O-methyl-D-ribofuranose.

Die Erfindung betrifft ferner ein Verfahren zur Herstellung der Zucker, die eine Isocyaniddichlorid-Gruppe an eines der nicht-anomeren C-Atome des Zuckermoleküls gebunden enthalten; das Verfahren ist dadurch gekennzeichnet, daß man in die Lösung von Zuckern der Formel I, die anstelle der Isocyaniddichlorid-Gruppe eine Isonitril-Gruppe aufweisen, in einem inerten organischen Lösungsmittel bei Temperaturen von -60 bis +40°C, vorzugsweise -30 bis +30°C solange langsam Chlor einleitet, bis IR-spektroskopisch im Reaktionsgemisch keine Isonitril-Gruppe mehr nachweisbar ist; dies ist im allgemeinen der Fall, wenn 1 Mol Chlor je Mol Isocyangruppen (-N≡C-Gruppen) in die Lösung eingeleitet wurde. Solche Zuckerisocyanide sind zum Teil bekannt (siehe z.B. J.C.S.Perkin I, 1980, 2657) oder können aus entsprechenden Aminozuckern durch Formylierung der Aminogruppe und anschließende Dehydratisierung des Formamids (siehe Synthesis 1985 , 400) auf einfache Weise hergestellt werden. Die Aminozucker kommen entweder natürlich vor und/oder können nach in der Zuckerchemie üblichen Methoden aus bekannten Zuckern hergestellt werden.

Als Vertreter der im erfindungsgemäßen Verfahren als Ausgangsverbindungen zu verwendenden Zuckerisonitrile seien beispielsweise genannt:

2-Isocyano-2.6-di-deoxy-1.3.4-tri-O-acetyl-D-glucopyranose;
2-Isocyano-2-deoxy-1-O-methyl-3.4.6-tri-O-benzoyl-D-mannopyranose;
2-Isocyano-2-deoxy-1-O-methyl-3-O-methansulfonyl-4.6-O-benzyliden-D-altropyranose;
3-Isocyano-3-deoxy-1.2-O-isopropyliden-5.6-O-cyclohexyliden-α-D-glucofuranose;
3-Isocyano-3-deoxy-1.2-O-isopropyliden-5.6-O-methyliden-α-D-allofuranose;
2-Isocyano-2-deoxy-1.3.4.6-tetra-O-acetyl-β-D-glucopyranose;
6-Isocyano-6-deoxy-1.2-O-isopropyliden-3.5-di-O-acetyl-α-D-glucofuranose;
6-Isocyano-6-deoxy-1-O-methyl-2.3.4-tri-O-acetyl-α-D-glucopyranose;
6-Isocyano-6-dexoy-1,2-O-isopropyliden-3,5-di-O-benzyl-α-D-glucofuranose;
3-Isocyano-3-deoxy-1.2-O-isopropyliden-5.6-O-isopropyliden-α-D-glucofuranose;
4-Isocyano-4-deoxy-1-O-methyl-2.3.6-tri-O-benzyl-α-D-glucopyranose;
3-Isocyano-3-deoxy-1.2-O-isopropyliden-5.6-O-isopropyliden-α-D-allofuranose sowie
6-Isocyano-2.6-di-deoxy-2-chloro-1-O-methyl-3.4-di-O-acetyl-D-mannopyranose und
5-Isocyano-5-deoxy-2.3-O-isopropyliden-1-O-methyl-D-ribofuranose.

Als inerte, d.h. sich unter den Reaktionsbedingungen nicht verändernde organische Lösungsmittel seien beispielsweise genannt chlorierte aromatische Kohlenwasserstoffe wie Chlorbenzol, Dichlorbenzol und Trichlorbenzol; nitrierte aromatische Kohlenwasserstoffe wie Nitrobenzol und halogenierte aliphatische Kohlenwasserstoffe wie Chloroform, Tetrachlorkohlenstoff, Tetrachlorethan und Pentachlor ethan; bevorzugt werden halogenierte aliphatische Kohlenwasserstoffe, insbesondere Chloroform und Tetrachlorkohlenstoff verwendet.

Die Lösungsmittel werden im allgemeinen in einer solchen Menge angewendet, daß auf 1 Gew.-Teil Isocyanid 1 bis 20, vorzugsweise 2 bis 12 Gew.-Teile Lösungsmittel entfallen.

Das erfindungsgemäße Verfahren wird im allgemeinen bei Normaldruck durchgeführt. Es ist aber auch möglich, das Verfahren bei einem Unter-oder Überdruck (beispielsweise im Druckbereich von 0,5 bis 10 bar) durchzuführen. Der Verlauf der Chlorierung kann IR-spektroskopisch verfolgt werden (Verschwinden der NC-Bande). Erfahrungsgemäß ist die Umsetzung nach Einleiten der für die Chlorierung erforderlichen Menge Chlor (1 Mol $Cl_2$ je Mol NC-Gruppe) beendet.

Das erfindungsgemäße Verfahren kann beispielsweise wie folgt durchgeführt werden:

Das Zucker-Isocyanid wird im Lösungsmittel gelöst. Die Lösung wird auf die gewünschte Reaktionstemperatur gebracht; anschließend wird unter starkem Rühren so lange Chlor in die Lösung eingeleitet, bis die Isonitril-Bande im IR-Spektrum einer Probe der Lösung verschwunden ist. Danach wird das Lösungsmittel abdestilliert und das Isocyaniddichlorid in an sich bekannter Weise, z.B. durch Umkristallisieren des Rückstandes, isoliert.

Die erfindungsgemäßen Zucker, die die Isocyaniddichlorid-Gruppe an eines der nicht-anomeren C-Atome des Zuckermoleküls gebunden enthalten, sind wertvolle Zwischenprodukte für die Herstellung von biologisch aktiven Verbindungen wie sie z.B. in den Japanischen Anmeldungen JA-A-76/075 072, JA-A-81/103 197 und JA-A-81/103 196 und der US-PS 3 575 959 beschrieben sind.

Beispiel 1

In die Lösung von 12,68 g (26,8 mmol) 4-Isocyano-4-deoxy-2.3.6-tri-O-benzyl-1-O-methyl-α-D-glucopyranose in 150 ml Chloroform wird bei -15°C langsam Chlor eingeleitet. Die Reaktion wird IR-spektroskopisch verfolgt. Nach dem Verschwinden der N≡C-Bande wird das Einleiten des Chlors abgebrochen. Das Lösungsmittel wird bei 0°C entfernt. Der Rückstand wird in 100 ml Cyclohexan in der Siedehitze gelöst. Die Lösung wird mit Aktivkohle geklärt. Aus dem Filtrat scheiden sich beim Stehen über Nacht Verunreinigungen ab. Die klare Cyclohexanphase wird abdekantiert und bis zur Gewichtskonstanz eingeengt.

Es werden 12,6 g (= 86,5 % der Theorie) 4-Dichlormethylenimino-4-deoxy-2.3.6-tri-O-benzyl-1-O-methyl-α-D-glucopyranose in Form eines farblosen Öls erhalten.

IR(Film) 1655 $cm^{-1}$ ≙ N = 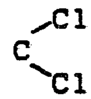

Elementaranalyse:

| | C | H | N (%) |
|---|---|---|---|
| ber: | 64 | 5,7 | 2,57 |
| gef: | 64,7 | 5,7 | 2,5 |

Beispiel 2

In die Lösung von 30 g (84,75 mmol) 2-Isocyano-2-deoxy-1.3.4.6-tetra-O-acetyl-β-D-glucopyranose in 300 ml Chloroform wird bei -5 bis -10°C so lange Chlor eingeleitet, bis sich IR-spektroskopisch keine Isonitrilgruppe nachweisen läßt. Anschließend wird das Lösungsmittel bei -10°C entfernt. Der Rückstand wird aus 300 ml Cyclohexan umkristallisiert.

Es werden 21,6 g (= 60 % der Theorie) 2-Dichlormethylenimino-2-deoxy-1.3.4.6-tetra-O-acetyl-β-D-glucopyranose in Form farbloser Kristalle vom Fp.: 124-125°C erhalten.

IR (KBr:) 1660 $cm^{-1}$ ≙ N = 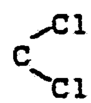

Elementaranalyse:

| | C | H | N | % |
|---|---|---|---|---|
| ber: | 42,06 | 4,44 | 3,27 | |
| gef: | 42,3 | 4,4 | 3,2 | |

Beispiel 3

In die Lösung von 3,1 g (11,52 mmol) 3-Isocyano-3-deoxy-1.2.5.6-di-O-isopropyliden-α-D-glucofuranose in 25 ml Chloroform wird bei -15°C langsam so lange Chlor eingeleitet, bis sich IR-spektroskopisch keine Isonitrilgruppe mehr nachweisen läßt. Anschließend wird das Lösungsmittel bei -10 bis 0°C entfernt. Der Rückstand wird mit 50 ml n-Hexan verrührt; dabei geht das Isocyaniddichlorid in Lösung und etwas polymeres Isonitril bleibt ungelöst zurück. Die Lösung wird vom Ungelösten abdekantiert und bei Raumtemperatur, zuletzt im Hochvakuum, bis zur Gewichtskonstanz eingeengt.

Es werden 3,68 g (= 93,95 % der Theorie) 3-Dichlormethylenimino-3-deoxy-1.2.5.6-di-O-isopropyliden-α-D-glucofuranose in Form eines farblosen Öls erhalten.

IR (Film): 1655 $cm^{-1}$ ≙ 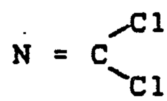

Elementaranalyse:

| | C | H | N % |
|---|---|---|---|
| ber: | 46 | 5,6 | 4,1 |
| gef: | 47 | 5,6 | 4,0 |

Beispiel 4

In die Lösung von 6,15 g (22,8 mmol) 3-Isocyano-3-deoxy-1.2.5.6-di-O-isopropyliden-α-D-allofuranose wird bei -30°C so lange Chlor eingeleitet, bis sich IR-spektroskopisch keine Isonitrilgruppe mehr nachweisen läßt. Der Rückstand wird in 150 ml n-Hexan gelöst; die Lösung wird mit Aktivkohle geklärt und anschließend im Vakuum bis zur Gewichtskonstanz eingeengt.

Es werden 6 g (= 77,4 % der Theorie) 3-Dichlormethylenimino-3-deoxy-1.2.5.6-di-O-isopropyliden-α-D-allofuranose in Form farbloser Kristalle vom Fp.: 68°C erhalten.

IR (KBr): 1660 $cm^{-1}$ ≙ 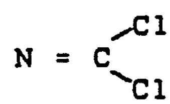

Elementaranalyse:

| | C | H | N % |
|---|---|---|---|
| ber: | 46 | 5,6 | 4,1 |
| gef: | 46 | 5,6 | 4,2 |

Beispiel 5

In die Lösung von 4,6 g (11,25 mmol) 6-Isocyano-6-deoxy-1,2-O-isopropyliden-3,5-di-O-benzyl-α-D-glucofuranose in 50 ml Methylenchlorid wird bei -20°C solange Chlor eingeleitet, bis sich IR-spektroskopisch keine Isonitrilgruppe mehr nachweisen läßt. Anschließend wird das Lösungsmittel im Vakuum destillativ abgetrennt. Der Rückstand wird in 50 ml n-Hexan gelöst; die Lösung wird mit Aktivgkohle geklärt und anschließend im Vakuum bis zur Gewichtskonstanz eingeengt.

Es werden 4,9 g (= 90,74 % der Theorie) 6-Dichlormethylenimino-6-deoxy-1,2-O-isopropyliden-3,5-di-O-benzyl-α-D-glucofuranose in Form eines leicht gelblich gefärbten Öls erhalten.

IR (Film): 1660 $cm^{-1}$ ≙ 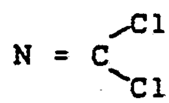

Elementaranalyse:

| | C | H | N |
|---|---|---|---|
| ber: | 60 % | 5,62 % | 2,92 % |
| gef: | 60,1 % | 5,6 % | 2,9 % |

**Ansprüche**

1. Eine Isocyaniddichlorid-Gruppe aufweisende Zucker, dadurch gekennzeichnet, daß sie die Isocyaniddichlorid-Gruppe an eines der nicht-anomeren C-Atome des Zuckermoleküls gebunden enthalten.

2. Eine Isocyaniddichlorid-Gruppe aufweisende Zucker gemäß Anspruch 1, dadurch gekennzeichnet, daß sie der Formel

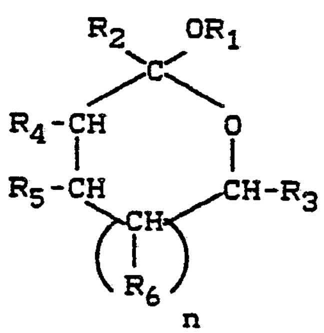 I

entsprechen, in der
n 0 oder 1,
$R_1$ eine Schutzgruppe und
$R_2$ Wasserstoff, Methyl, Ethyl oder $-CH_2-R_7$ ist,
$R_3$ für Wasserstoff, Methyl, Ethyl, $-CH_2-R_8$ oder $-C(H)R_9-CH_2-R_{10}$ steht und
$R_4$, $R_5$, $R_6$, $R_7$, $R_8$, $R_9$ und $R_{10}$ unabhängig voneinander für Wasserstoff, Halogen, eine geschützte OH-Gruppe, eine Isocyaniddichlorid-Gruppe oder den Rest eines cyclischen Acetals der Formel

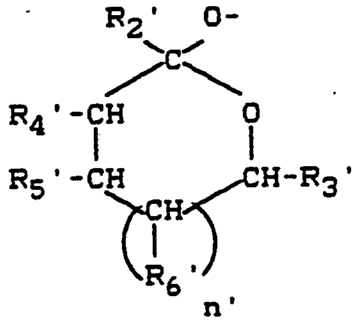

II

stehen, in der
n′ 0 oder 1,
$R_2'$ Wasserstoff, Methyl, Ethyl oder $-CH_2-R_7'$ ist
$R_3'$ für Wasserstoff, Methyl, Ethyl, $-CH_2-R_8'$ oder $-C(H)R_9'-CH_2-R_{10}'$ steht,
$R_4'$, $R_5'$, $R_6'$, $R_7'$, $R_8'$, $R_9'$, und $R_{10}'$ unabhängig voneinander ist Wasserstoff, Halogen, eine geschützte OH-Gruppe oder den Rest eines cyclischen Acetals der Formel II stehen,
mit der Maßgabe,
daß nur einer der Reste $R_4$-$R_{10}$ eine Isocyaniddichlorid-Gruppe ist, daß höchstens einer der Reste $R_4$-$R_{10}$ und höchstens einer der Reste $R_4'$-$R_{10}'$ für den Rest eines cyclischen Acetals der Formel II steht und daß mindestens einer der Reste $R_4$-$R_{10}$ und mindestens einer der Reste $R_4'$-$R_{10}'$ eine geschützte OH-Gruppe ist.

3. Eine Isocyaniddichlorid-Gruppe aufweisender Zucker gemäß Anspruch 1, dadurch gekennzeichnet, daß in Formel I
n 0 oder 1,
$R_1$ eine Schutzgruppe und
$R_2$ Wasserstoff oder $-CH_2-R_7$ ist,
$R_3$ für Wasserstoff, Methyl, $-CH_2-R_8$ oder $-C(H)R_9-CH_2-R_{10}$ steht und
$R_4$, $R_5$, $R_6$, $R_7$, $R_8$, $R_9$ und $R_{10}$ unabhängig voneinander Wasserstoff, eine geschützte OH-Gruppe oder eine Isocyaniddichloridgruppe bedeuten,
mit der Maßgabe, daß nur einer der Reste $R_4$-$R_{10}$ eine Isocyaniddichloridgruppe ist und mindestens zwei der Reste $R_4$-$R_{10}$ eine geschützte OH-Gruppe sind.

4. Eine Isocyaniddichlorid-Gruppe aufweisende Zucker gemäß Anspruch 1, dadurch gekennzeichnet, daß in Formel I
n 0 oder 1
$R_1$ eine Schutzgruppe und
$R_2$ Wasserstoff ist,
$R_3$ für Wasserstoff, $-CH_2-R_8$ oder $-C(H)R_9-CH_2-R_{10}$ steht und
$R_4$, $R_5$, $R_6$, $R_8$, $R_9$ und $R_{10}$ unabhängig voneinander eine geschützte OH-Gruppe oder eine Isocyanatdichlorid-Gruppe bedeuten,
mit der Maßgabe, daß nur einer der Reste $R_4$, $R_5$, $R_6$ $R_8$, $R_9$ oder $R_{10}$ eine Isocyaniddichlorid-Gruppe ist.

5. Verfahren zur Herstellung von Zuckern, die eine an ein nicht-anomeres C-Atom des Zuckermoleküls gebundene Isocyaniddichlorid-Gruppe aufweisen, dadurch gekennzeichnet, daß man in die Lösung von Zuckern, die eine an ein nicht-anomeres C-Atom des Zuckermoleküls gebundene Isonitril-Gruppe aufweisen, in einem inerten organischen Lösungsmittel bei Temperaturen von -60 bis +40° so lange Chlor einleitet, bis IR-spektroskopisch keine Isonitril-Gruppe mehr nachweisbar ist.

6. Verfahren gemäß Anspruch 5, dadurch gekennzeichnet, daß man die Chlorierung bei Temperaturen von -30 bis + 30°C vornimmt.

7. Verfahren nach Ansprüchen 5 oder 6, dadurch gekennzeichnet, daß als Zucker, die eine an ein nicht-anomeres C-Atom des Zuckermoleküls gebundene Isonitril-Gruppe aufweisen, Zucker gemäß einem der Ansprüche 2, 3 oder 4 verwendet, die anstelle der Isocyaniddichlorid-Gruppe eine Isonitril-Gruppe aufweisen.